# EUROPÄISCHE PATENTSCHRIFT

(11) **EP 2 417 556 B1**
(45) Veröffentlichungstag und Bekanntmachung des Hinweises auf die Patenterteilung: **22.03.2017**
(21) Anmeldenummer: 09796597.4
(22) Anmeldetag: 07.10.2009
(51) Int. Cl.: G06F 21/71, G06F 19/00, G06F 21/72, H04L 29/06

(54) **DATENVERARBEITUNGSGERÄT MIT ZERTIFIZIERBARER VERSCHLÜSSELUNG**
COMPUTER SYSTEM WITH CERTIFIABLE ENCRYPTION MODULE
SYSTÈME DE COMPUTATION AVEC MODULE D'ENCHIFFREMENT CERTIFIABLE

(30) Priorität: 08.10.2008 DE 102008050739
(43) Veröffentlichungstag der Anmeldung: 15.02.2012
(73) Patentinhaber: Sommer, Ralf, 63867 Johannesberg (DE)
(72) Erfinder: Sommer, Ralf, 63867 Johannesberg (DE)
(74) Vertreter: Baumann, Rüdiger Walter
(86) Internationale Anmeldenummer: PCT/DE2009/001389
(87) Internationale Veröffentlichungsnummer: WO 2010/040341

(56) Entgegenhaltungen:
- WO-A2-01/69556
- DE-A1- 10 311 327
- US-A1- 2003 126 434
- US-A1- 2004 098 625
- US-A1- 2005 283 620

## Beschreibung

Die Erfindung bezieht sich auf ein Datenverarbeitungsgerät zur Verarbeitung sicherheitskritischer Datenelemente eines Datenstromes, bestehend aus einem ersten Teil, der Sicherheitsbaugruppe, und einem zweiten Teil, der Verarbeitungsbaugruppe, in denen Datenelemente verarbeitbar sind, die in wenigstens einem für alle Datenelemente gleichen Teil, dem Zuordnungsblock, einen sicherheitskritischen Bestandteil enthalten.

Auf aktuellem Stand der Technik werden z. B. die Käufe von Aktienpaketen, die Verwaltung von Ersatzteilen für Waffensysteme, Atomkraftwerke, Erdölbohrinseln und Flugzeuge aber auch Vorgänge von weniger hohem Wert wie z. B. die Bezahlung von Verzehrrechnungen mit Kreditkarten über den Austausch von Datenelementen abgewickelt. Dadurch wird das Risiko unerwünschten oder gar unerlaubten Zugriffs auf die Datenelemente immer größer. Meist nur lästig ist die Zusendung von unerwünschten Werbesendungen. Tragisch ist es, wenn durch unzulässige Manipulation z. B. ein sicherheitskritisches Ersatzteil gegen ein scheinbar gleiches, aber mit einem unsichtbaren Fehler behaftetes ausgetauscht wird und dadurch ein vermeidbarer Unfall entsteht. Ganz konkret bezifferbare Schäden entstehen dann, wenn durch unberechtigten Zugriff auf Kreditkarten nicht autorisierte Ausgaben getätigt werden.

Um möglichen Sicherheitslücken einen möglichst effizienten Riegel vorzuschieben, wird deshalb mit verschiedenen Verfahren versucht, den direkten Zugriff auf unverschlüsselte, sicherheitskritische Datenelemente bei der Datenverarbeitung zu vermeiden, wofür auf aktuellem Stand der Technik zahlreiche Verschlüsselungsverfahren bekannt sind.

Die DE 603 15 726, Lotspiech beschreibt ein Verfahren, mit dem bei einer Anfrage eines Kunden per Internet an einen Lieferanten die Offenlegung seiner persönlichen Daten innerhalb des Lieferantenunternehmens auf denjenigen - sehr kleinen - Personenkreis beschränkt wird, der so genannte Sitzungsschlüsselblöcke an registrierte Kunden vergibt. Der Kunde kontaktiert den Lieferanten nur unter einem Sitzungsschlüsselblock, den er durch sog. Einheitenschlüssel, die er vom Lieferanten erhält, berechnen kann.

Der Nachteil dieser Methode ist, dass in einem ersten Schritt eine Anmeldung des Kunden nötig ist und in einem zweiten Schritt die Übergabe der Einheitenschlüssel. Erst im dritten Schritt kann der Kunde seine Transaktion starten.

Eine Überwachung der Sicherheit dieses Verfahrens durch eine dritte Organisation ist sehr aufwendig, da innerhalb des Lieferantenunternehmens nur sehr mühsam rekonstruierbar ist, wo ein Schritt dieses Verfahrens tatsächlich abgelaufen ist und welche Person und/oder welcher Rechner diese Schritte ausgeführt hat.

Die WO 2008/036914 erläutert, wie die Verarbeitung eines Datenelementes in einem EDV-System dadurch anonymisiert wird, dass im Server der EDV zumindest der Identifizierungsteil eines Datenelementes durch einen Token ersetzt wird. Der Token ist ein Platzhalter oder ein Symbol, das stellvertretend für den vollständigen Inhalt oder die zugeordnete Identität steht.

US 2004/0098625 A1 beschreibt ein Kommunikationsnetzwerk als Verbindungsstelle zwischen einem Internet Terminal eines Verbrauchers auf der einen Seite und einem Service Provider auf der anderen Seite, über das der Verbraucher eine persönliche Anfrage an den Service Provider richtet. In einem Logik-Bereich werden sämtliche Identifikationsdaten des Verbrauchers durch ein "Alias" ersetzt und vollständig im Logik-Bereich gespeichert und die Anfrage des Verbrauchers anonymisiert durch den Service-Provider bearbeitet. Nach der Bearbeitung wird im Logik-Bereich das "Alias" wieder durch die Identifikationsdaten des Verbrauchers ersetzt.

Ein wesentlicher Nachteil ist, dass diese Verschlüsselung im Server des EDV-Systems durchgeführt werden soll. Ein Server ist meist ein komplexes Gebilde aus mehreren Teilen im Zentrum des EDV-Systems, das aber die Ausführung von Aufgaben in andere Anlagen-teile auslagern kann. Deshalb ist auch bei dieser Anmeldung nicht genau festlegbar, wo ein Schritt dieses Verfahrens tatsächlich abgelaufen ist und welche Anlagenteile diese Schritte ausgeführt haben.

Eine weitere Einschränkung der Sicherheit bei der beschriebenen Verschlüsselung über einen Token ist, das der Zugriff auf den Server innerhalb eines Unternehmens zwar fast immer gewissen Restriktionen unterliegen wird, die aber keinem allgemein akzeptierten Standard unterliegen und die - versehentlich oder vorsätzlich - unterlaufen werden können. Ein Zugriff auf die Kodierung und Dekodierung der Token - z. B. von einer eigentlich nicht berechtigten Workstation auf den Server - kann also nicht mit der gebotenen Sicherheit ausgeschlossen werden.

Deshalb ist auch für die beschriebene Verschlüsselung über einen Token eine Überwachung der Sicherheit durch eine dritte Organisation sehr aufwendig, da das gesamte EDV-System überprüft werden muss.

Die Patentanmeldung US 2004/ 00 986 25 A1 beschreibt ein Kommunikationsnetzwerk als Verbindungsstelle zwischen einem Internet Terminal eines Verbrauchers auf der einen Seite und einem Service Provider auf der anderen Seite, über das der Verbraucher eine persönliche Anfrage an den Service Provider richtet. In einem Logik-Bereich werden sämtliche Identifikationsdaten des Verbrauchers durch ein "Alias" ersetzt und vollständig im Logik-Bereich gespeichert und die Anfrage des Verbrauchers anonymisiert vom Service-Provider bearbeitet. Nach der Bearbeitung wird im Logikbereich das "Alias" wieder durch die Identifikationsdaten des Verbrauchers ersetzt.

Der wesentliche Nachteil dieses Systems ist, dass das Risiko einer missbräuchlichen Verwendung der persönlichen Daten des Verbrau chers nur vom Service Provider in die Logik-Einheit verschoben ist, denn dort sind alle persönlichen Daten der Verbraucher in einer sog. "Verbraucher-Datenbank" gespeichert. Die Daten sind dort vollständig vorhanden und können als für sich aussagefähiges Paket entwendet und missbraucht werden.

Ein weiterer, wesentlicher Nachteil ist, dass für die Einfügung des "Alias" in den Datensatz kein Verfahren angegeben ist, das eine absolute Sicherheit gegen einen unberechtigten Zugriff über die Datenleitungen bietet.

Auf diesem Hintergrund hat sich die Erfindung die Aufgabe gestellt, für die Bearbeitung von sicherheitskritischen Datenelementen eine relativ einfache und kostengünstig zu realisierende Anonymisierung zu entwickeln, die einen missbräuchlichen Zugang zu den vollständigen sicherheitskritischen Teilen eines Datenelementes über eine einzige Baugruppe ausschließt und die für die Anonymisierung der sicherheitskritischen Daten ein Verfahren vorgibt, das für jeden Datensatz anders ist und die Anonymisierung eines bestimmten Datensatzes aus der Kenntnis anderer Anonymisierungsvorgänge nicht ableitbar ist.

Als Lösung präsentiert die Erfindung, dass die kritischen Informationen eines Datenelementes in der einen Richtung des Datenstromes aufgespalten werden, und ein erster Teil der kritischen Information in der Sicherheitsbaugruppe zusammen mit dem Interimscode speicherbar ist und der zweite Teil der kritischen Information zusammen mit dem Interimscode an die Verarbeitungsbaugruppe weiterleitbar ist und in der anderen Richtung des Datenstromes die kritischen Informationen in der Sicherheitsbaugruppe wieder miteinander verschmolzen werden.

Ein wesentliches Merkmal der Erfindung ist also, dass der sicherheitskritische Bestandteil - auch kritische Information genannt - eines jeden Datenelementes in zwei Teile aufgespalten wird. Nur der erste Teil wird in der Sicherheitsbaugruppe gespeichert. Der zweite Teil des sicherheitskritischen Bestandteils wird an die Verarbeitungsbaugruppe - auch als "nachfolgendes System" bezeichnet - weitergegeben. Der zweite Teil wird also unverändert und zusammen mit den übrigen Bestandteilen des Datenelementes weitergereicht.

Dadurch können weder aus der Sicherheitsbaugruppe für sich alleine noch aus der nachfolgenden Verarbeitungsbaugruppe als einzelner Baugruppe die alle sicherheitskritischen Daten entnommen werden, da sie weder in der Sicherheitsbaugruppe noch in der Verarbeitungsbaugruppe vollständig existieren.

Datenelemente, die keine sicherheitskritischen Bestandteile, also auch keinen Zuordnungsblock enthalten, werden von der Sicherheitsbaugruppe unverändert an die Verarbeitungsbaugruppe weiter gegeben.

Der Interimscode kann bei gezielter Absicht mit dem Diebstahl der Sicherheitsbaugruppe zwar entwendet werden, ist aber nutzlos, da er nur einen für sich aussagelosen Teil der sicherheitskritischen Daten enthält. Ein Zugriff auf die Daten ist nur den Personen möglich, die sowohl einen Zugriff auf die Sicherheitsbaugruppe und die darin gelagerte erste Teilliste als auch auf die zweite Teilliste in der nachgelagerte Verarbeitung haben. Ein Zusammenfügen der getrennt gespeicherten Daten kann ein Unberechtigter nicht durch das Entschlüsseln einer Verschlüsselung aus einer sicheren Ferne über eine Datenleitung erreichen, da es keine Verschlüsselung gibt.

Dank dieser Beschränkung ist die ganz überwiegende Anzahl der normalerweise an einem Datenverarbeitungsprozess beteiligten Personen von einem Zugriff auf die vollständigen sicherheitskritischen Daten ausgeschlossen.

Die Sicherheitsbaugruppe arbeitet ähnlich wie ein Katalysator, bei dem die Reaktion in Abhängigkeit von der Richtung unterschiedlich ist: In der einen Richtung werden kritische Informationen aufgespalten und den einzelnen Teilen der Interimscode als ein Identifikator zugewiesen. Ein Teil der Information wird in der Sicherheitsbaugruppe zusammen mit dem Interimscode gespeichert, während der andere Teil der Information zusammen mit dem Interimscode an nachgelagerte Systeme weitergeleitet wird. Jedes der beiden Teile der Information ist für sich isoliert betrachtet unkritisch.

In der anderen Richtung werden die Informationen in der Sicherheitsbaugruppe wieder miteinander verschmolzen, so dass die kritische Information wieder vorhanden ist und weitergeleitet werden kann.

Eine wesentliche Eigenschaft der Erfindung ist also, dass kritische Daten in einem Datenelement durch die Sicherheitsbaugruppe aufgespalten werden, so dass jeder aufgespaltene Teil für sich alleine keine kritische Information darstellt. Auch der Interimscode, der in jedem Datenelement eingesetzt wird, lässt außerhalb der Sicherheitsbaugruppe keine Wiederherstellung oder Ableitung des ersetzten Teils zu.

Nachgelagerte Systeme können meist ganz ohne oder mit nur geringfügigen Änderungen das Datenelement mit dem Interimscode verarbeiten.

Erst in der Sicherheitsbaugruppe und unter Verwendung des Interimscodes kann der zu einem vollständigen Datenelement zugehörige, abgespaltene Teil identifiziert und wieder mit dem restlichen Datenelement zur ursprünglichen, kritischen Information vereinigt werden.

Wenn der Code in einem anonymisiert hergestellten Chip in die Sicherheitsbaugruppe eingebracht wird, ist es sogar denkbar, dass er nur durch den sehr hohen Aufwand des Abschleifens vom Chip und dessen Auswertung unter einem Mikroskop bestimmbar ist.

Auch wenn ein anderes Verfahren zum Anbringen des Codes verwandt wird, ist es ein prinzipieller Vorteil der erfindungsgemäßen Sicherheitsbaugruppe, dass sich der sicherheitskritische Bereich nur auf einen sehr kleinen Abschnitt der gesamten Hardware des Datenverarbeitungsgerätes beschränkt. Dadurch ist nicht nur ein einfacher Wechsel möglich, sondern auch ein relativ aufwendiges Sicherheitssystem bei dessen Herstellung und dessen Implementierung.

Eine Grundbedingung ist die möglichst weitgehende Trennung zwischen der Sicherheitsbaugruppe und der Verarbeitungsbaugruppe im Datenverarbeitungsgerät. Idealerweise sollten beide nur durch eine Datenschnittstelle miteinander verbunden werden.

Durch diese einfach zu erfüllenden Voraussetzungen eignet sich das erfindungsgemäße System sogar für den Einsatz in mobilen Systemen, wie z. B. Verkaufsstationen an Bord von Flugzeugen.

In einer besonders vorteilhaften Ausführungsform ist die Sicherheitsbaugruppe unabhängig von der Verarbeitungsbaugruppe betreibbar, kann also inselartig überprüft werden. Dabei muss die Eingabe eines Datenelementes und der Ersatz des Zuordnungsblocks im Datenelement durch einen Interimscode und das Aus- und Einlesen eines Datenelementes mit Interimscode und der Rücktausch des Interimscodes in einem Datenelement gegen den ursprünglich vorhandenen Zuordnungsblock möglich sein. Damit kann die gesamte Funktionalität der Sicherheitsbaugruppe außerhalb eines Datenverarbeitungsgerätes getestet werden.

Das eröffnet die Möglichkeit, die Sicherheitsbaugruppe getrennt von den übrigen Teilen des Datenverarbeitungsgerätes durch eine unabhängige Organisation überprüfen und zertifizieren zu lassen.

Dabei sind verschiedene Ausführungsformen für den Schlüssel zur Umwandlung der Zuordnungsblöcke in einem Interimscode möglich. Im einfachsten Fall ist es ein einziger Schlüssel, der für die Umwandlung in den Interimscode ebenso wie die Rückwandlung des Interimscodes in den ursprünglichen Zuordnungsblock angewendet wird. Alternativ können jedoch mehrere Schlüssel vorhanden sein. Dann muss im Interimscode die Information darüber enthalten sein, welcher der vorhandenen Schlüssel für die Rückwandlung eines Interimscodes in den ursprünglichen Zuordnungsblock anzuwenden ist.

In der ganz überwiegenden Mehrzahl der Fälle werden die Datenelemente und die Codierung elektronisch übertragen. Die Erfindung schließt jedoch ausdrücklich die Hinterlegung des Schlüsselcodes in einem mechanischen Element mit in den Anspruch ein. Eine solche Lösung ist auf aktuellem Stand der Technik unüblich und derzeit nur bei der Zugangsberechtigung von Gebäuden als Haustürschlüssel üblich. Es ist also durchaus denkbar, dass auch für andere Systeme ein Code z. B. durch Einkerbungen in einem Metallstück hinterlegt wird. Durch eine optische Abtastung wäre ein verschleißfreies Auslesen des Codes möglich. Prinzipiell nicht unähnliche Leseköpfe sind z. B. aus DVD-Abspielgeräten millionenfach bekannt.

Zur Erhöhung der Sicherheit der Verschlüsselung können in der Sicherheitsbaugruppe auch eine Reihe von verschiedenen Interimscodes gespeichert werden. Von einem Zufallsgenerator wird ein einziger dieser verschiedenen Interimscodes ausgewählt, um den Zuordnungsblock eines Datenelementes zu ersetzen. In einem Speicher innerhalb der Sicherheitsbaugruppe ist abspeicherbar, welcher Interimscode welchen Zuordnungsblock ersetzt hat. Diese Information ist bei der Rückwandlung eines Interimscodes in den ursprünglichen Zuordnungsblock aus dem Speicher abrufbar.

Diese Kombination des Interimscodes mit einem Zuordnungsblock kann in der Sicherheitsbaugruppe mit einer weiteren Verschlüsselung gespeichert werden.

Für den Fall, dass sicherheitsrelevante Datenelemente ungeplanter und unzulässiger Weise dennoch an der Sicherheitsbaugruppe vorbei in die Verarbeitungsbaugruppe gelangt sind, ist es hilfreich, dass jeder Interimscode ein Kennzeichen enthält, das ihn eindeutig von jedem Zuordnungsblock unterscheidet. Dadurch wird es möglich, dass in der Verarbeitungsbaugruppe nur solche Daten zur Verarbeitung zugelassen werden, die sich als ordnungsgemäß mit einem Interimscode versehen qualifiziert haben.

Ein erfindungsgemäßes Datenverarbeitungsgerät ist für die verschiedensten Anwendungsfälle sicherheitssteigernd einzusetzen. Ein hoch interessantes Anwendungsgebiet ist die Verarbeitung von Kreditkartennummern. Dabei kann der Zuordnungsblock alle Stellen einer Kreditkartennummer ab der siebten Stelle bis zur fünftletzten Stelle jeweils einschließlich umfassen. Die ersten sechs Stellen und die letzten vier Stellen werden also unverschlüsselt übertragen. Wenn die Kreditkartennummer z.B. 16-stellig ist, dann sind entsprechend der vorgenannten Definition die Stellen 7 bis 12 der Zuordnungsblock.

Bei dieser Anwendung ist es ein wesentlicher Vorteil, dass die hardwaremäßig getrennte Sicherheitsbaugruppe nach dem Payment-Card-Industry-Date-Security-Standard PCIDSS zertifizierbar ist. Dadurch wird der enorme Kostenaufwand der Zertifizierung eines gesamten Datenverarbeitungssystems erspart. Insbesondere ist es möglich, auch mobile Außenstationen dieses Systems zertifiziert zu betreiben. Ein Anwendungsbeispiel sind die mobilen Verkaufswagen im Gang eines Verkehrsflugzeugs.

Eine Kreditkartennummer ist i.d.R, stets einer einzigen Person zugeordnet und muss deshalb mit der gleichen Sorgfalt und dem gleichen hohen Datenschutz behandelt werden wie eine Personenkennziffer.

Ein erfindungsgemäßes Datenverarbeitungsgerät, das den hohen Standard der Verarbeitung von Kreditkartennummern erfüllt, kann deshalb auch höchst effizient zur Verarbeitung von anderen Personenkennziffern eingesetzt werden. Mögliche Beispiele sind Kennkarten in Gesundheitssystemen, die Krankheitsverläufe des Individuums abspeichern. Durch erfindungsgemäße Datenverarbeitungsgeräte kann eine solche Karte auch für mögliche Gesundheitsüberprüfung durch neutrale Organisationen genutzt werden, ohne dass das Risiko besteht, eventuell dabei ermittelte Risikoinformationen einem unberechtigten Zugriff aussetzen zu müssen.

Das hohe Sicherheitsniveau der erfindungsgemäßen Datenverarbeitung gewährleistet z.B. auch im Umgang mit Daten über die finanzielle Position einer Person einen sehr wirkungsvollen Schutz. Dazu zählen u.a. der Kontostand, die finanzielle Bonität, die Zahlungsverpflichtungen und der Kreditrahmen einer natürlichen Person oder einer Institution.

Weit weniger spektakulär ist die Anwendung z. B. eines maschinenlesbaren Führerscheins als Altersnachweis in einem Verkaufsautomat, der erst oberhalb einer bestimmten Altersgrenze Waren frei gibt. Hier schließt ein erfindungsgemäßes Datenverarbeitungsgerät die Speicherung der Personendaten aus und bewahrt damit den Betreiber des Verkaufsautomaten vor möglichen Vorwürfen der missbräuchlichen Weitergabe von Benutzerdaten.

Ein erfindungsgemäßes Datenverarbeitungsgerät kann auch für das Handling von Datenelementen benutzt werden, die Gegenständen zugeordnet sind. Anwendungsfälle sind z. B. Baugruppen oder Ersatzteile, die eine zentrale oder kritische Funktion in sehr großen und/sehr gefährlichen Einrichtung sind wie z. B. im militärischen Waffensystemen, Atomkraftwerken, Erdölbohrinseln, Flugzeugen, Staudämmen, Unfalldatenspeichern, Seuchenforschungsinstituten, Schließanlagen eines Gebäudes, Geldtransportsystemen, Sondermüllverarbeitungen oder Großrechnern verantworten und ausführen.

In weiteren Ausführungsvarianten kann ein erfindungsgemäßes Datenverarbeitungsgerät mit zusätzlichen Sicherungseinrichtungen versehen werden. Ein Ansatzpunkt dafür ist der Interimscode und seine Erzeugung, Speicherung, sowie seine Zuordnung beim Abspalten und bei der Rückführung des Zuordnungsblockes.

In diesem Sinne schlägt die Erfindung als eine Alternative vor, dass der Interimscode auf ein besonderes Signal hin umgeschlüsselt wird. Dieses Signal für die Umschlüsselung kann z. B. durch einen festen Zeitgeber generiert werden. Andere Auslöser für das Umschlüsselungssignal sind u.a. Vorgänge, die auf eine missbräuchliche Nutzung und/oder eine Manipulation an der Sicherheitsbaugruppe hinweisen können, wie z. B. eine Unterbrechung in der Spannungsversorgung in der Sicherheitsbaugruppe, eine Demontage des Gehäuses oder eine Beschleunigung oberhalb eines gewissen Grenzwertes, also Schläge auf die Baugruppe oder eine Demontage des gesamten Gerätes.

Sinnvoll ist es auch, wenn die Sicherheitsbaugruppe ein Verfahren zum Schutz der Datensicherung der gespeicherten Daten von Interimscode und Zuordnungsblöcken unterstützt.

In einer weiteren Variante enthält die Sicherheitsbaugruppe mehrere Untersicherheitsbaugruppen mit der gleichen Funktion wie eine nicht segmentierte Sicherheitsbaugruppe ohne die vorgenannten Untersicherheitsbaugruppen. Eine zusätzliche Synchronisationsbaugruppe wacht dann darüber, dass der Strom der eintreffenden Datenelemente gleichmäßig auf alle funktionstüchtigen Untersicherheitsbaugruppen verteilt wird. Beim Ausfall einer Untersicherheitsbaugruppe gehen auf diese Weise keine Daten verloren. Ein weiterer Vorteil ist, dass alle Untersicherheitsbaugruppen stets gleichmäßig ausgelastet werden. In dieser Ausführungsvariante unterstützt die Sicherheitsbaugruppe also die Synchronisation mehrerer Untersicherheitsbaugruppen mit dem Ziel der Ausfallsicherheit und Lastverteilung.

Als eine weitere Option wird im Interimscode hinterlegt, von welcher der Untersicherheitsbaugruppen der Zuordnungsblock ersetzt worden ist. Dadurch kann der "Herausgeber" eines Interimscodes identifiziert werden.

Als eine weitere Ausstattungsoption kann in der Sicherheitsbaugruppe gewählt werden, ob identischen Zuordnungsblöcken oder identischen Datenelementen entweder stets der gleiche Interimscode oder jeweils ein neuer Interimscode zugeordnet werden kann. Wenn identischen Zuordnungsblöcken oder identischen Datenelementen immer der gleiche Interimscode zugeordnet wird, so wird das auch als "Verfahren 1:n" bezeichnet. Wenn jedem Zuordnungsblock oder Datenelement immer ein eindeutig neuer Interimscode zugeordnet wird, so wird diese Methode als "Verfahren 1:1" bezeichnet.

Beim Verfahren 1:n werden z. B. für alle derzeit existierenden, schätzungsweise mehrere hundert Millionen von 16-stelligen Kreditkartennummern der Speicherbedarf auf maximal eine Million Interimscodes in der Sicherheitsbaugruppe reduziert. In der Praxis reduziert sich die Menge der notwendigen Interimscodes bei dieser Zuordnungsvariante noch weiter, da bestimmte Nummernbereiche für Zuordnungsblöcke nicht benutzt werden oder durch Prüfsummenverfahren eingeschränkt sind.

Im allgemeinsten Fall ist der Zuordnungsblock in der Sicherheitsbaugruppe ohne weitere Verschlüsselung gespeichert, da er für sich alleine i.d.R. keine sicherheitskritische Information darstellt. So ist z. B. die Speicherung der mittleren Stellen einer Kreditkartennummer als Zuordnungsblock gemäß dem PCIDSS-Standard unverschlüsselt möglich, da dieser Zuordnungsblock weder eine gültige Kreditkartennummer darstellt noch daraus eine gültige Kreditkartennummer abgeleitet werden kann.

Ebenso stellt die Speicherung eines Namens als Zuordnungsblock keine sicherheitskritische Information dar, da dieser keinem Datenelement ohne Decodierung des jeweiligen Interimscodes zugeordnet werden kann.

Da die Kenntnis des jeweiligen Interimscodes den Zugang zu allen sicherheitskritischen Informationen ermöglicht, sobald nur ein Exemplar der Sicherheitsbaugruppe zur Verfügung steht, sind weitere Sicherheitsmaßnahmen zur Absicherung des Interimscodes höchst sinnvoll.
Als einen sehr einfachen Zusatzschutz schlägt die Erfindung eine verschlüsselte Speicherung der Interimscodes vor, sodass die Zuordnung der Interimscodes in einem Datenelement zu einem gespeicherten Zuordnungsblock nur über den Codierschlüssel in der Sicherheitsbaugruppe möglich ist. Dadurch ist bei Entwendung der Daten aus der Sicherheitsbaugruppe keine Zuordnung der Interimscodes der Datenelemente zu den gespeicherten Zuordnungsblöcken möglich.

Wenn z. B. bei der Bearbeitung von Kreditkartennummern der Interimscode jedes Zuordnungsblockes verschlüsselt in der Sicherheitsbaugruppe gespeichert ist, und die Daten aus der Sicherheitsbaugruppe entwendet werden, so können dennoch die Interimscodes aus den Kreditkartennummern keinem Zuordnungsblock zugeordnet werden, da die Entschlüsselung des gespeicherten Interimscodes nur mit dem Codierschlüssel der Sicherheitsbaugruppe möglich ist.

Ein erfindungsgemäßes Datenverarbeitungsgerät kann die verschiedensten Datenelemente verarbeiten, unabhängig davon, ob sie der Teil einer Datenbanktabelle oder ein Teil eines Schriftstückes sind und unabhängig davon, ob sie als Word-File, PDF-File, HTML-File oder XML-File eingegeben werden.

Weitere Optionen sind, dass der Schlüssel für die Verschlüsselung für die Kombination eines Interimscodes mit einem Zuordnungsblock erst bei der Inbetriebnahme und/oder bei jedem Hochfahren der Sicherheitsbaugruppe eingelesen wird. Dadurch ist die Sicherheitsbaugruppe aus dem Verantwortungsbereich des Herstellers herausgenommen. Frei nach dem Entscheid des Betreibers kann die Sicherheitsbaugruppe mit einer neuen Verschlüsselung versehen werden.

Zu einer weiteren Verbesserung des Sicherheitsniveaus kann der Schlüssel für die Verschlüsselung in mehrere Teile aufgeteilt werden, deren Verwaltung verschiedene Personen zu verantworten haben. Zusätzlich kann für die Eingabe dieser Verschlüsselungsteile bei der Inbetriebnahme oder bei jedem Hochfahren der Sicherheitsbaugruppe eine bestimmte Reihenfolge für die Eingabe vorgegeben werden.

Der Schlüssel für die Verschlüsselung ist nicht als Ganzes einer einzigen Person bekannt. Außerhalb der Sicherheitsbaugruppe darf der Schlüssel nur in Teilen von unterschiedlichen Personen verwaltet werden. Erst in der Sicherheitsbaugruppe wird der Schlüssel für die Verschlüsselung aus den unterschiedlichen Teilen zusammengesetzt. Damit ist ausgeschlossen, dass eine einzelne Personen den Zugriff auf die vollständige Verschlüsselung des Schlüssels erhält und somit die Daten der Sicherheitsbaugruppe entwenden und alle verschlüsselten Interimscodes dekodieren könnte.

Als Alternative kann die Verschlüsselung des Schlüssels auch Teil der Hardware der Sicherheitsbaugruppe sein.

Im Folgenden sollen weitere Einzelheiten und Merkmale der Erfindung anhand eines Beispiels näher erläutert werden. Dieses soll die Erfindung jedoch nicht einschränken, sondern nur erläutern. Es zeigt in schematischer Darstellung:

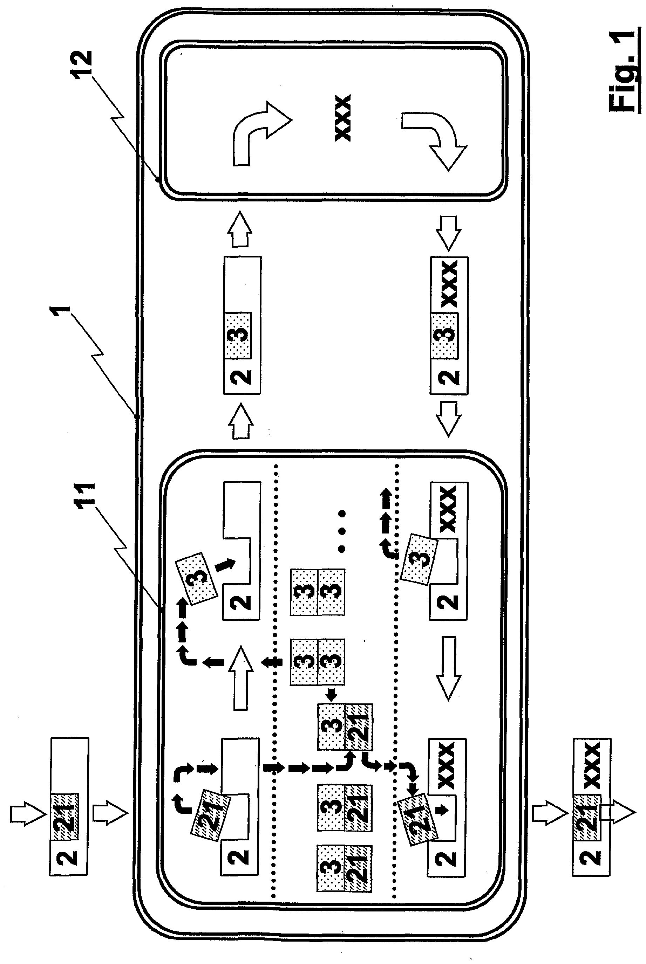

- Figur 1: Weg eines Datenelementes durch ein erfindungsgemäßes Datenverarbeitungsgerät

In **Figur 1** ist ein erfindungsgemäßes Datenverarbeitungsgerät 1 schematisch dargestellt. Dabei sind die Hardware-Baugruppen, nämlich das Datenverarbeitungsgerät 1 und die darin enthaltene Sicherheitsbaugruppe 11 und die Verarbeitungsbaugruppe 12, durch Blöcke mit abgerundeten Ecken und doppelter Umrandung gekennzeichnet. In dieser Hardwareumgebung ist der Weg eines Datenelementes 2 dargestellt, wobei jedes Datenelement 2 durch ein längliches Rechteck symbolisiert ist und ein Ausschnitt dieses Rechteckes, der durch eine Schraffur gekennzeichnet ist, den sicherheitskritischen Zuordnungsblock 21 des Datenelementes 2 darstellt.

In Figur 1 ist oben links ein Datenelement 2 eingezeichnet, der in seinem Zuordnungsblock 21 sicherheitskritische Daten mitbringt. Durch doppelte Pfeile ist der Weg des Datenelementes 2 durch das Datenverarbeitungsgerät 1 markiert. Innerhalb des Datenverarbeitungsgerätes 1 tritt er zuerst in die Sicherheitsbaugruppe 11 ein. Dort wird der Zuordnungsblock 21 entnommen - was durch einen gestrichelten Pfeil gekennzeichnet ist - und dann innerhalb der Sicherheitsbaugruppe 11 einem Interimscode 3 zugeordnet. Jeder Interimscode 3 ist durch ein Rechteck symbolisiert, welches die gleiche Größe wie der Zuordnungsblock 21 hat, jedoch gepunktet ist.

In der dargestellten Ausführungsform wird der Interimscode 3 einem Speicher von per Zufallsgenerator erzeugten Interimscodes 3 entnommen. Durch die symbolische Darstellung als zwei identische, aufeinander liegende Rechtecke wird angedeutet, dass der Interimscode einmal die Stelle des Zuordnungsblockes im Datenelement 2 einnimmt und zum zweiten der gleiche Interimscode 3 gemeinsam mit dem soeben entnommenen Zuordnungsblock 21 in einem Speicher der Sicherheitsbaugruppe 11 hinterlegt wird. Er "wartet" im Speicher der Sicherheitsbaugruppe 11 bis "sein" zugehöriger Datenelement 2 nach dem Weg durch die Verarbeitungsbaugruppe 12 wieder in die Sicherheitsbaugruppe 11 zurückkehrt und dort den Interimscode 3 wieder "abgibt" und stattdessen seinen dort "geparkten" Zuordnungsblock 21 wieder aufnimmt, sodass er nunmehr - im bearbeiteten Zustand und mit seinem ursprünglichen Zuordnungsblock 21 versehen - die Sicherheitsbaugruppe 11 verlassen kann. Der dabei benutze Interimscode 3 "verbleibt" im Speicher der Sicherheitsbaugruppe und kann bei einem weiteren, eintreffenden Datenelement 2 wieder verwendet werden.

In Figur 1 wird durch die grafische Darstellung der obersten Zeile der Sicherheitsbaugruppe 11 symbolisiert, dass dort ein jedes Datenelement 2 seinen Zuordnungsblock 21 gegen den Interimscode 3 austauscht und dessen Zuordnung zum jeweiligen Zuordnungsblock 21 in der Sicherheitsbaugruppe gespeichert wird, sodass nach erfolgter Verarbeitung der sichere Rücktausch möglich ist.

In Figur 1 ist die ganz deutliche, hardwaremäßige Trennung zwischen der Sicherheitsbaugruppe 11 und der Verarbeitungsbaugruppe 12 durch zwei unterschiedliche Baugruppen als je ein Rechteck mit abgerundeten Ecken und doppelter Umrandung gekennzeichnet. Beide Baugruppen sind nur durch zwei Datenschnittstellen miteinander verbunden. Auf der einen verlässt das noch nicht bearbeitete Datenelement 2 die Sicherheitsbaugruppe 11 und tritt in die Verarbeitungsbaugruppe 12 ein. Dort wird er bearbeitet, was in Figur 1 durch drei Kreuze auf dem Datenelement 2 gekennzeichnet ist. Die Bearbeitung kann ein sehr umfangreicher Vorgang sein. Auch die Verarbeitungsbaugruppe 11 kann ein großes Rechnersystem sein. Auf der zweiten Datenschnittstelle tritt er dann wieder in die Sicherheitsbaugruppe 11 ein. Dort wird er identifiziert und dann sein Interimscode 3 entnommen und wiederum durch den ursprünglichen Zuordnungsblock 21 ersetzt. Nach dieser Prozedur verlässt das nunmehr bearbeitete Datenelement 2 die Sicherheitsbaugruppe.

In Figur 1 ist im Interesse einer einfachen Symbolisierung diejenige Ausführungsvariante einer erfindungsgemäßen Sicherheitsbaugruppe 11 gewählt, bei der gemäß Unteranspruch 7 in der Sicherheitsbaugruppe 11 eine Reihe von verschiedenen Interimscodes 3 gespeichert ist und von einem Zufallsgenerator einer dieser verschiedenen Interimscodes 3 auswählbar ist, um den Zuordnungsblock 21 eines Datenelementes 2 zu ersetzen. In einem Speicher innerhalb der Sicherheitsbaugruppe 11 ist dann abspeicherbar, welcher Interimscode 3 welchen Zuordnungsblock 21 ersetzt hat, sodass bei der Rückwandlung eines Interimscodes 3 der ursprüngliche Zuordnungsblock 21 aus dem Speicher abrufbar ist.

Diese beiden Speicherfunktionen sind in Figur 1 innerhalb der Sicherheitsbaugruppe 11 im Mittleren der drei Streifen symbolisiert, die durch gepunktete Linien voneinander getrennt sind: In der rechten Hälfte des mittleren Streifens sind mehrere gepunktete Rechtecke zu sehen, die die bereit gehaltenen Interimscodes 3 darstellen. Es sind zwei Rechtecke übereinander gezeichnet, um zu symbolisieren, dass der gleiche Code zum einen in jedem Datenelement 2 den ursprünglichen Zuordnungsblock 21 ersetzt und ein gleicher Interimscode 3 in dem weiteren Speicher der Sicherheitsbaugruppe 11 zusammen mit dem eben entnommenen Zuordnungsblock 21 gespeichert wird.

Deshalb sind in der linken Hälfte der mittleren Zeile der Sicherheitsbaugruppe 11 gepunktete Rechtecke über schraffierten Rechtecken eingezeichnet. Sie symbolisieren die im zweiten Speicher der Sicherheitsbaugruppe 11 hinterlegte Kombination aus Zuordnungsblock 21 und Interimscode 3 für alle Datenelemente 2, die augenblicklich in der Verarbeitungsbaugruppe 12 verarbeitet werden und/oder sich auf dem Weg dort hin oder von dort befinden.

### Bezugszeichenliste

- 1: Datenverarbeitungsgerät
- 11: Sicherheitsbaugruppe im Datenverarbeitungsgerät 1
- 12: Verarbeitungsbaugruppe im Datenverarbeitungsgerät 1
- 2: Datenelement zur Verarbeitung im Datenverarbeitungsgerät 1
- 21: Zuordnungsblock des Datenelementes 2, der die sicherheitskritische Zuordnung enthält
- 3: Interimscode, ersetzt während der Datenverarbeitung vorübergehend den Zuordnungsblock 21

## Patentansprüche

1. Datenverarbeitungsgerät 1 zur Verarbeitung sicherheitskritischer Datenelemente 2 eines Datenstromes, bestehend aus
- einem ersten Teil, der Sicherheitsbaugruppe 11, und
- einem zweiten Teil, der Verarbeitungsbaugruppe 12,
in denen Datenelemente 2 verarbeitbar sind, die in wenigstens einem für alle Datenelemente 2 gleichen Teil, dem Zuordnungsblock 21, eine kritische Information enthalten,
wobei
in der Sicherheitsbaugruppe 11 jeder Zuordnungsblock 21 des Datenelementes 2 durch je einen Interimscode 3 ersetzt wird, der ausschließlich in der Sicherheitsbaugruppe 11 codierbar ist und
- das mit dem Interimscode 3 versehene Datenelement 2 in der Verarbeitungsbaugruppe 12 verarbeitet wird und
- bei jedem bearbeiteten und jedem unbearbeiteten Datenelement 2 ausschließlich in der Sicherheitsbaugruppe 11 der Interimscode 3 wieder durch den ursprünglichen Zuordnungsblock 21 ersetzbar ist **dadurch gekennzeichnet, dass** die kritischen Informationen eines Datenelementes 2 in der einen Richtung des Datenstromes aufgespalten werden, und
- ein erster Teil der kritischen Information in der Sicherheitsbaugruppe 11 zusammen mit dem Interimscode 3 speicherbar ist und
- der zweite Teil der kritischen Information zusammen mit dem Interimscode 3 an die Verarbeitungsbaugruppe 12 weiterleitbar ist
und
- in der anderen Richtung des Datenstromes die kritischen Informationen in der Sicherheitsbaugruppe 11 wieder miteinander verschmolzen werden und
- in der Sicherheitsbaugruppe 11 eine Reihe von verschiedenen Interimscodes 3 gespeichert ist, und
- von einem Zufallsgenerator einer dieser verschiedenen Interimscodes auswählbar ist, um den Zuordnungsblock 21 eines Datensatzes 2 zu ersetzen und
- in einem Speicher innerhalb der Sicherheitsbaugruppe abspeicherbar ist, welcher Interimscode 3 welchen Zuordnungsblock 21 ersetzt hat und
- bei der Rückwandlung eines Interimscodes der ursprünglichen Zuordnungsblock 21 aus dem Speicher abrufbar ist
und
- die Sicherheitsbaugruppe 11 und die Verarbeitungsbaugruppe 12 nur durch eine Datenschnittstelle miteinander verbunden sind.

2. Datenverarbeitungsgerät 1 nach einem der vorhergehenden Ansprüche, **dadurch gekennzeichnet, dass** die Sicherheitsbaugruppe 11 unabhängig von der Verarbeitungsbaugruppe 12 betreibbar ist und dabei
- die Eingabe wenigstens eines Datenelementes 2 und
- der Ersatz des Zuordnungsblocks 21 im Datenelement 2 durch einen Interimscode 3 und
- das Auslesen und das Einlesen eines Datenelementes 2 mit Interimscode 3 und
- der Rücktausch des Interimscodes 3 in einem Datenelement 2 gegen den ursprünglich vorhandenen Zuordnungsblock 21 möglich ist.

3. Datenverarbeitungsgerät 1 nach einem der vorhergehenden Ansprüche, **dadurch gekennzeichnet, dass** innerhalb der Sicherheitsbaugruppe 11 die Kombination eines Interimscodes 3 und eines Zuordnungsblockes 21 unter einer Verschlüsselung speicherbar ist.

4. Datenverarbeitungsgerät 1 nach einem der vorhergehenden Ansprüche, **dadurch gekennzeichnet, dass** das Datenelement 2 eine Kreditkartennummer ist, wobei der Zuordnungsblock 21 alle Stellen einer Kreditkartennummer ab der siebten Stelle bis zur fünftletzten Stelle jeweils einschließlich umfassen kann oder alle der nach dem jeweils gültigen PCI DSS-Standard als sicherheitskritisch eingestuften Stellen.

5. Datenverarbeitungsgerät 1 nach einem der vorhergehenden Ansprüche, **dadurch gekennzeichnet, dass** die Sicherheitsbaugruppe 11 nach dem Payment Card Industry Data Security Standard - PCIDSS - zertifizierbar ist.

6. Datenverarbeitungsgerät 1 nach einem der vorhergehenden Ansprüche, **dadurch gekennzeichnet, dass** das Datenelement 2 eine Personenkennziffer ist oder.
- den Kontostand und/oder
- die finanzielle Bonität und/oder
- die Zahlungsverpflichtungen und/oder
- den Kreditrahmen
einer natürlichen Person oder einer Institution enthält oder einem Gegenstand zugeordnet ist, der
Teil eines
- militärischen Waffensystems oder
- eines Atomkraftwerkes oder
- einer Erdölbohrinsel oder
- eines Flugzeuges oder
- eines Staudamms oder
- eines Unfalldatenspeichers oder
- eines Seuchenforschungsinstitutes oder
- einer Schließanlage für Gebäude oder
- eines Geldtransportsystems oder
- einer Sondermüllverarbeitung oder
- eines Großrechners ist.

7. Datenverarbeitungsgerät 1 nach einem der vorhergehenden Ansprüche, **dadurch gekennzeichnet, dass** die Sicherheitsbaugruppe 11 eine Einrichtung zum Schutz der gespeicherten Interimscodes 3 und der gespeicherten Zuordnungsblöcke 21 enthält.

8. Datenverarbeitungsgerät 1 nach einem der vorhergehenden Ansprüche, **dadurch gekennzeichnet, dass** die in der Sicherheitsbaugruppe 11 verarbeiteten Datenelemente 2
- ein Teil einer Datenbank-Tabelle oder
- ein Teil einer Datei wie z.B. Word oder PDF oder
- ein Teil eines Datenstromes wie z.B. HTML oder XML sind.

9. Datenverarbeitungsgerät 1 nach Anspruch 3, **dadurch gekennzeichnet, dass** der Schlüssel für die Verschlüsselung für die Kombination eines Interimscodes 3 und eines Zuordnungsblockes 21
- bei der Inbetriebnahme und/oder
- jedem Hochfahren
der Sicherheitsbaugruppe 11 eingelesen wird.

10. Datenverarbeitungsgerät 1 nach Anspruch 9, **dadurch gekennzeichnet, dass** der Schlüssel für die Verschlüsselung in mehrere Teile aufgeteilt ist, deren Verwaltung verschiedene Personen verantworten.

11. Datenverarbeitungsgerät 1 nach den vorhergehenden Ansprüchen 9 und 10, **dadurch gekennzeichnet, dass** bei der Inbetriebnahme und/oder bei jedem Hochfahren für die Eingabe der Teile des Schlüssels für die Verschlüsselung eine bestimmte Reihenfolge vorgegeben ist.

12. Datenverarbeitungsgerät 1 nach Anspruch 11, **dadurch gekennzeichnet, dass** der Schlüssel für die Verschlüsselung Teil der Hardware der Sicherheitsbaugruppe 11 ist.

## Claims

1. A data processing device 1 for processing security-critical data elements 2 of a data stream, consisting of:
- a first part, the security module 11, and
- a second part, the processing module 12,
wherein data elements 2 which contain critical information in at least one part which is the same for all data elements 2, the allocation block 21, can be processed in these parts,
wherein,
in the security module 11, each allocation block 21 of the data element 2 is replaced by a respective interim code 3, which can only be coded in the security module 11, and
- the data element 2 which is provided with the interim code 3 is processed in the processing module 12, and
- for each processed and each unprocessed data element 2, the interim code 3 can be replaced again by the original allocation block 21 only in the security module 11,
**characterized in that**
the critical information of a data element 2 is divided in one direction of the data stream, and
- a first part of the critical information can be stored together with the interim code 3 in the security module 11, and
- the second part of the critical information can be routed to the processing module 12 together with the interim code 3, and
- in the other direction of the data stream, the critical information can be fused back together in the security module 11,
and
- a series of different interim codes 3 is stored in the security module 11, and
- one of these different interim codes can be selected by a random generator in order to replace the allocation block 21 of a data set 2, and
- which interim code 3 has replaced which allocation block 21 can be stored in a memory inside the security module, and
- when an interim code is converted back, the original allocation block 21 can be called from the memory,
and
- the security module 11 and the processing module 12 are only connected to each other by one data interface.

2. The data processing device 1 according to one of the preceding claims, **characterized in that** the security module 11 can be operated independently of the processing module 12, and it is possible to
- input at least one data element 2, and
- to replace the allocation block 21 in the data element 2 with an interim code 3, and
- to output and input a data element 2 with an interim code 3, and
- to re-exchange the interim codes 3 in a data element 2 for the allocation block 21 which was originally present.

3. The data processing device 1 according to one of the preceding claims, **characterized in that** the combination of an interim code 3 and an allocation block 21 can be stored in the security module 11 in encrypted form.

4. The data processing device 1 according to one of the preceding claims, **characterized in that** the data element 2 is a credit card number, wherein the allocation block 21 can include all digits of a credit card number from the seventh digit to the fifth-to-last digit, or all the digits which are considered security-critical according to the currently-valid PCIDSS standard.

5. The data processing device 1 according to one of the preceding claims, **characterized in that** the security module 11 can be certified according to the Payment Card Industry Data Security Standard - PCIDSS.

6. The data processing device 1 according to one of the preceding claims, **characterized in that** the data element 2 is a personal identification number, or contains
- the account balance and/or
- the financial standing and/or
- the payment obligations and/or
- the credit limit
of a natural person or an institution, or
is assigned to an object which
is part of
- a military weapons system, or
- a nuclear power plant, or
- an oil rig, or
- an airplane, or
- a dam, or
- an accident data memory, or
- a disease or research institute, or
- a locking system for buildings, or
- a money transport system, or
- a hazardous waste processing, or
- a mainframe.

7. The data processing device 1 according to one of the preceding claims, **characterized in that** the security module 11 includes a means for protecting the stored interim codes 3 and the stored allocation blocks 21.

8. The data processing device according to one of the preceding claims, **characterized in that** the data elements 2 processed in the security module 11 are
- a portion of a database table, or
- a portion of a file such as a Word or .pdf file, or
- a portion of a data stream, such as HTML or XML.

9. The data processing device 1 according to claim 3, **characterized in that** the key for the encryption for the combination of an interim code 3 and an allocation block 21 is input
- at initial start-up, and/or
- at each start-up
of the security module 11.

10. The data processing device 1 according to claim 9, **characterized in that** the key for the encryption is divided into multiple parts, and different people are responsible for the management of these parts.

11. The data processing device 1 according to one of the previous claims 9 and 10, **characterized in that** a specific sequence is prespecified for inputting the parts of the key for the encryption upon initial start-up and/or at each start-up.

12. The data processing device 1 according to claim 11, **characterized in that** the key for the encryption is part of the hardware of the security module 11.

## Revendications

1. Système de computation 1 pour le traitement d'éléments de données 2 critiques pour la sécurité d'un flux de données, composé
- d'un premier élément, le module de sécurité 11, et
- d'un deuxième élément, le module de traitement 12,
dans lesquels des éléments de données 2 peuvent être traités, qui contiennent une information critique dans au moins un élément identique pour tous les éléments de données 2, le bloc d'affectation 21,
dans lequel
dans le module de sécurité 11, chaque bloc d'affectation 21 de l'élément de données 2 est remplacé par respectivement un code temporaire 3, qui peut exclusivement être codé dans le module de sécurité 11 et
- l'élément de données 2 doté du code temporaire 3 est traité dans le module de traitement 12 et
- pour chaque élément de données 2 traité et chaque élément de données 2 non traité, le code temporaire 3 peut être remplacé à nouveau par le bloc d'affectation d'origine 21 exclusivement dans le module de sécurité 11
**caractérisé en ce que**
les informations critiques d'un élément de données 2 sont scindées dans une direction du flux de données, et
- une première partie de l'information critique peut être enregistrée dans le module de sécurité 11 conjointement avec le code temporaire 3 et
- la deuxième partie de l'information critique peut être transférée conjointement avec le code temporaire 3 au module de traitement 12 et
- dans l'autre direction du flux de données, les informations critiques sont fusionnées à nouveau dans le module de sécurité 11 et
- une série de différents codes temporaires 3 est enregistrée dans le module de sécurité 11, et
- un de ces différents codes temporaires peut être sélectionné par un générateur aléatoire pour remplacer le bloc d'affectation 21 d'un jeu de données 2 et
- il peut être enregistré dans une mémoire à l'intérieur du module de sécurité quel code temporaire 3 a remplacé quel bloc d'affectation 21 et
- lors de la reconversion d'un code temporaire, le bloc d'affectation d'origine 21 peut être appelé de la mémoire
et
- le module de sécurité 11 et le module de traitement 12 sont reliés l'un à l'autre uniquement par une interface de données.

2. Système de computation 1 selon l'une quelconque des revendications précédentes, **caractérisé en ce que** le module de sécurité 11 peut être exploité indépendamment du module de traitement 12 et ce faisant
- la saisie d'au moins un élément de données 2 et
- le remplacement du bloc d'affectation 21 dans l'élément de données 2 par un code temporaire 3 et
- la lecture et l'écriture d'un élément de données 2 avec code temporaire 3 et
- l'échange du code temporaire 3 dans un élément de données 2 contre le bloc d'affectation 21 initialement présent est possible.

3. Système de computation 1 selon l'une quelconque des revendications précédentes, **caractérisé en ce que** la combinaison d'un code temporaire 3 et d'un bloc d'affectation 21 peut être enregistrée chiffrée à l'intérieur du module de sécurité 11.

4. Système de computation 1 selon l'une quelconque des revendications précédentes, **caractérisé en ce que** l'élément de données 2 est un numéro de carte de crédit, dans lequel le bloc d'affectation 21 peut englober tous les chiffres d'un numéro de carte de crédit du septième chiffre au cinquième dernier chiffre respectivement compris ou tous les chiffres considérés comme critiques pour la sécurité selon la norme PCI-DSS respectivement valable.

5. Système de computation 1 selon l'une quelconque des revendications précédentes, **caractérisé en ce que** le module de sécurité 11 peut être certifié selon la norme de sécurité de l'industrie des cartes de paiement - PCIDSS.

6. Système de computation 1 selon l'une quelconque des revendications précédentes, **caractérisé en ce que** l'élément de données 2 est un identifiant personnel ou comporte
- le solde bancaire et/ou
- la solvabilité financière et/ou
- les obligations de paiement et/ou
- le plafond de crédit
d'une personne physique ou d'une institution ou
est affecté à un objet qui
fait partie
- d'un système d'armes militaire ou
- d'une centrale nucléaire ou
- d'une plateforme pétrolière ou
- d'un avion ou
- d'un barrage ou
- d'un enregistreur de données d'accident ou
- d'un institut de recherche épidémiologique ou
- d'un système de fermeture pour bâtiments ou
- d'un système de transport de fonds ou
- d'un système de traitement de systèmes spéciaux ou
- d'un ordinateur central.

7. Système de computation 1 selon l'une quelconque des revendications précédentes, **caractérisé en ce que** le module de sécurité 11 comporte un équipement de protection des codes temporaires enregistrés 3 et des blocs d'affectation enregistrés 21.

8. Système de computation 1 selon l'une quelconque des revendications précédentes, **caractérisé en ce que** les éléments de données 2 traités dans le module de sécurité 11 sont
- une partie d'un tableau de base de données ou
- une partie d'un fichier par exemple Word ou PDF ou
- une partie d'un flux de données par exemple HTML ou XML.

9. Système de computation 1 selon la revendication 3, **caractérisé en ce que** la clé pour l'enchiffrement pour la combinaison d'un code temporaire 3 et d'un bloc d'affectation 21 est écrite
- lors de la mise en service et/ou
- à chaque démarrage
du module de sécurité 11.

10. Système de computation 1 selon la revendication 9, **caractérisé en ce que** la clé pour l'enchiffrement est divisée en plusieurs parties, dont la gestion est de la responsabilité de différentes personnes.

11. Système de computation 1 selon les revendications précédentes 9 et 10, **caractérisé en ce que** lors de la mise en service et/ou à chaque démarrage, un ordre donné est prédéfini pour la saisie des éléments de la clé pour l'enchiffrement.

12. Système de computation 1 selon la revendication 11, **caractérisé en ce que** la clé pour l'enchiffrement fait partie du matériel du module de sécurité 11.
